# EUROPEAN PATENT SPECIFICATION

(11) **EP 3 324 964 B1**
(45) Date of publication and mention of the grant of the patent: **11.08.2021**
(21) Application number: 16831202.3
(22) Date of filing: 25.07.2016
(51) Int. Cl.: A61K 31/4425, A61K 31/44, A61P 25/08

(54) **AMPHIPHILIC PYRIDINIUM COMPOUNDS TO TREAT EPILEPSY AND OTHER DISORDERS OF THE NERVOUS SYSTEM**
AMPHIPHILE PYRIDINIUMVERBINDUNGEN ZUR BEHANDLUNG VON EPILEPSIE UND ANDEREN ERKRANKUNGEN DES NERVENSYSTEMS
COMPOSÉS DE PYRIDINIUM AMPHIPHILES DESTINÉS À TRAITER L'ÉPILEPSIE ET D'AUTRES TROUBLES DU SYSTÈME NERVEUX

(30) Priority: 24.07.2015 US 201514808650
(43) Date of publication of application: 30.05.2018
(73) Proprietor: Pollard, Bette, Silver, Potomac, MD 20854 (US)
(72) Inventor: Pollard, Bette, Silver, Potomac, MD 20854 (US)
(74) Representative: PATERIS Patentanwälte PartmbB
(86) International application number: PCT/US2016/043896
(87) International publication number: WO 2017/019619

(56) References cited:
- WO-A1-2014/209507
- US-A1- 2007 105 916
- US-A1- 2012 258 126
- US-A1- 2014 030 223
- US-A1- 2014 187 505
- US-A1- 2015 111 945
- US-A1- 2015 166 532
- US-A1- 2016 022 656
- SUSANNA TCHILIBON ET AL: "Amphiphilic pyridinium salts block TNF[alpha]/NF[kappa]B signaling and constitutive hypersecretion of interleukin-8 (IL-8) from cystic fibrosis lung epithelial cells", BIOCHEMICAL PHARMACOLOGY, vol. 70, no. 3, 1 August 2005 (2005-08-01) , pages 381-393, XP055554148, US ISSN: 0006-2952, DOI: 10.1016/j.bcp.2005.05.002
- YVONNE M. HART: "Management of epilepsy", MEDICINE - U K EDITION, vol. 40, no. 9, 1 September 2012 (2012-09-01), pages 477-483, XP055554180, GB ISSN: 1357-3039, DOI: 10.1016/j.mpmed.2012.06.003

## Description

### FIELD OF THE INVENTION

The present invention relates to the use of amphiphilic pyridinium salts and compounds, for the purpose of the treatment of seizure disorders such as epilepsy, temporal lobe epilepsy, drug resistant temporal lobe epilepsy, partial onset seizures, refractory partial seizures, tonic clonic seizures, and other types of seizure disorders, as well as other neurological disorders such as but not limited to migraines, neuralgia, such as but not limited to post herpetic neuralgia, neuronal cancers, obesity, anxiety disorders, schizophrenia, manic-depressive disorder, depression and major depressive disorder, and as an analgesic for relief from pain. It is also proposed that this drug can be used as an adjuvant therapy in combination with standard drugs to treat the disorder, such as standard anti-epileptic drugs (SAEDs). It is also described but does not belong to the invention that the amphiphilic pyridinium salts, either alone or in combination with digitoxin, are small molecule inhibitors of Ebola viruses (EBOVs) infection or Zika viruses (ZIKVs) infection. Further, disclosed is a method of treating or preventing infection by Zika Virus, or other arboviruses from the Flaviviridae family, or for treating or preventing Ebola virus or other viruses from the Filoviridae family, in a host that includes administering one or more cardiac glycosides in an amount effective for inhibiting arbovis infections in a host in need of inhibition of arbovirus infection.

### BACKGROUND OF THE INVENTION

Epilepsy is a group of neurological disorders characterized by epileptic seizures. It is a chronic condition of the brain. It is a complex chronic neurological disorder presented in a vast set of diseases that may not have any obvious cause, and is characterized by spontaneous recurrence surges of cortical nerve cell electrical activity in the brain resulting in unprovoked seizures. It is thought the prevalence is about 50 million people worldwide. The Centers for Disease Control and Prevention (CDC) estimate there are around 2.2 million people in the US with epilepsy. The incidence is about 48 of every 100,000 people. Thus, 150,000 people will develop epilepsy in their lifetime. Drug therapy remains ineffective for seizure control in about 30% of patients because either the drugs do not control the seizures or the patients cannot tolerate the side effects.

The seizures episodes can vary from brief and nearly undetectable to long periods of vigorous shaking. In epilepsy, seizures tend to recur and have no immediate underlying cause, while seizures that occur due to a specific cause are not deemed to represent epilepsy. The cause may be a result of brain injury, stroke, brain tumor, drug or alcohol abuse. Genetic mutations are linked to a small proportion of the disease. The diagnosis typically involves ruling out other conditions that might cause similar symptoms such as fainting, and determining if other causes are present such as alcohol withdrawal or electrolyte problems. This may be done by imaging the brain and performing blood tests. Epilepsy can often be confirmed with an electroencephalogram (EEG) but a normal test does not rule out the condition.

Seizures are controllable with medication in about 70% of cases. Some have seizures that do not respond to medication, surgery, neurostimulation or dietary changes, such as the Ketogenic diet. Not all cases are lifelong.

About 60% of seizures are convulsive. Generalized seizures affect both hemispheres of the brain, and account for 1/3 of the cases. Partial seizures (also called focal seizures), affect one hemisphere of the brain in 2/3 of cases, and may then progress to generalized seizures. The other 40% are non-convulsive. For example, there may be an absence seizure (petit mal), a decreased level of consciousness which usually lasts about 10 seconds. About 6% of people have seizures that are triggered by specific stimuli, such as flashing lights and sudden noises. Some seizures occur during sleep. Only about 25% of people with seizures have epilepsy.

Partial seizures are often preceded by an aura. They may include sensory (visual, hearing or small), psychic, autonomic, or motor phenomena. Jerking may start in a specific muscle group and spread to surrounding muscle groups. Non-consciously generated activities and simple repetitive movements like smacking of the lips may occur.

There are six main types of generalized seizures: tonic-clonic, tonic, clonic, myoclonic, absence, and atonic seizures. Generalized seizures all involve loss of consciousness and typically happen without warning.

Tonic-clonic seizures (grand mal) present with a contraction of the limbs followed by their extension along with arching of the back which lasts 10-30 seconds (the tonic phase). A cry may be heard. Then a shaking of the limbs occurs in unison (clonic phase). Tonic seizures produce constant contractions of the muscles. A person may turn blue from stoppage of breathing. After shaking stops, it may take 10-30 minutes (postictal state) for person to return to normal. There may be loss of bowel or bladder control. The tongue may be bitten.

Myoclonic seizures involve spasms of muscles in either a few areas or all over. Absence seizures can be subtle with only a slight turn of the head or eye blinking. Then the person returns to normal. Atonic seizures involve the loss of muscle activity for more than one second, typically on both sides of the body.

Temporal lobe epilepsy is a chronic neurological condition characterized by recurrent, unprovoked epileptic seizures which originate in the temporal lobe of the brain. They involve sensory changes, such as smelling an unusual odor, or a memory disturbance. The most common cause is mesial temporal sclerosis. Treatment is medication or surgery. Partial seizures account for about 60% of all adult cases. Temporal lobe epilepsy (TLE) is the single most common form of partial seizure.

There is mesial temporal lobe epilepsy (MTLE) arising in the hippocampus, the parahippocampal gyrus and the amygdala. The other more rare type, lateral temporal lobe (LTLE), arises in the neocortex at the outer (lateral) surface of the temporal lobe. Autosomal dominant Lateral Temporal Lobe Epilepsy (ADLTLE) is a rare hereditary condition.

Temporal lobe epilepsy and Drug resistant temporal lobe epilepsy is associated with a proinflammatory phenotype in the brain and blood, manifest by activation of the NFκB signaling pathway, and resulting in downstream elevation of proinflammatory cytokines and chemokines such as interleukin-1-beta (IL-1β), interleukin-8 (IL-8) and others (Pollard et al, 2013). Mounting evidence suggests that normal damage control processes in astrocytes and glial cells may contribute to a feed forward loop that promotes epileptic activity (Devinsky et al,2013; Eisenstein, 2014). Neuronally driven pathological electrical activity may activate the glial cells. Once activated, proinflammatory mediators secreted by the glia may initiate a signaling cascade in neurons that renders them more sensitive to glutamate-induced excitation (During and Spencer, 1993). The inflammatory response may also disrupt the blood-brain-barrier, releasing proinflammatory cytokines and chemokines into the general circulation (Librizzi L, et al. 2012).

The likely involvement of a proinflammatory phenotype for epilepsy has also been implicated in several other recent studies. For example, chronic stimulation of the vagus nerve has been shown to reduce the frequency of adverse events in refractory epilepsy (De Herdt et al, 2009). High levels of various proinflammatory mediators, including IL-8, have been found in surgical excisions of epileptic foci in brains from children with intractable epilepsy (Choi et al. 2009). Injections of kainic acid into the hippocampus to induce seizure activity in rats has resulted in elevated levels of proinflammatory mediators in the rat brain (Lauren et al 2010). In the case of neonatal seizures, elevated levels of proinflammatory mediators have been found in serum (Youn et al, 2012).

Amphiphilic pyridinium compounds have been shown to block TNFα/NFκB signaling and downstream interleukin-8 (IL-8) secretion from cells *in vitro* (Tchilibon et al. 2005). Optimal inhibitory activities were observed for MRS2481 and its optical isomer MRS2485. These compounds also block the neurotoxic calcium channels formed by amyloid beta peptide (Abeta[1-40]) in neuronal membranes, and protect neurons from Abeta[1-40] dependent cell death (Diaz JC, et al, 2009). Thus these amphiphilic pyridinium compounds have been considered from the vantage point of candidate Alzheimer's Disease drugs. Temporal lobe epilepsy and Drug resistant temporal lobe epilepsy have also been associated with some degree of cognitive impairment, as well as a proinflammatory phenotype.

Ebola viruses (EBOVs) and Zika viruses (ZIKVs) share affinity for AXL, a cell surface tyrosine receptor kinase, which they both use to gain entrance into host cells. This common affinity may also be the basis of a common vulnerability, should there be a way to safely interfere with AXL function. It is widely appreciated that EBOVs are responsible for lethal epidemics in West Africa, and are presently without either effective vaccines for prevention, or antiviral small molecules for therapy.

Arboviruses are a group of predominantly single-stranded+ RNA viruses, also termed flaviviridae, that are transmitted by arthropod vectors (Fauci AS and Moren DM, 2016). The arthropods responsible include mosquitos and ticks. Aedes aegypti is the primary mosquito species that transmits Zika virus, Dengue Fever (DENV; also known as "Break bone Fever"), Yellow fever and Chikungunya Virus. By contrast, the Culex mosquito species transmits Japanese Encephalitis Virus, Rift Valley Fever, and West Nile Virus (WNV).

Zika Virus (ZIKV) is the first mosquito-transmitted arbovirus shown to cause fetal abnormalities and microcephaly in unborn babies. Adults develop fever, rash, arthralgia and conjunctivitis, and can also develop Guillain-Barre syndrome. A candidate viral entry receptor, AXL, from the TAM phosphatidylserine receptor family, has been identified in the developing brain, and is highly expressed by radial glial cells, astrocytes, endothelial cells and microglia (Nowakowski TJ, et al, 2016). High levels of AXL expression are conserved in developing mouse and ferret cortex (Nowakowski et al, 2016). Cancer cells also express high levels of AXL, including breast (Meric et al, 2002), lung (Wimmel et al, 2001; Hamel et al, 2015), lymphomas and leukemias (Challier et al, 1996), colon (Craven et al, 1995), and prostate (Bansal et al, 2015).

The mosquito takes advantage of the fact that skin is a convenient site of entry for both ZIKV and dengue virus (Hamel et al, 2015). There, AXL is also the major candidate viral entry receptor in dermal fibroblasts, epidermal keratinocytes, immature dendritic cells, and lung epithelial cells. (Hamel R et al, 2015). Other minor candidate Zika receptors have been identified in a subset of cells from the epidermis, including adhesion factors DC-SIGN, TYRO3, and TIM-1 (Hamel et al, 2015).

Except for Yellow Fever, there are no commercial vaccines presently available for Zika, Dengue Fever or other arbovirus infections, nor are any available for Ebola virus or other Filviridae viruses. However, vaccines are effective only if the host has been vaccinated. A drug to prevent entry of ZIKV, or other arbovirus, into the host would treat those who were not vaccinated. Furthermore, there is a developing concern as to whether a previous infection with, or vaccinated against, one flavivirus might mediate antibody-dependent enhancement of a secondary severe infection (Haug et al, 2016). Therefore, a targeted antiviral against the entry mechanism for ZIKV would have additional advantages.

US 2007/0105916 A1 discloses amphiphilic pyridinium compounds for suppressing IL-8 secretion and production.

### SUMMARY OF THE INVENTION.

The present invention is directed to a pharmaceutical composition including amphiphilic pyridinium compounds for use in treating epilepsy and related neurological disorders characterized by epileptic seizures. The use of the amphiphilic pyridinium compounds for treating viral infections caused by EBOVs or ZIKVs or Marburg virus is also described but does not belong to the invention.

A method of inhibiting Zika virus infection in a host is disclosed but does not belong to the invention, wherein the method comprises administering one or more cardiac glycosides in an amount effective for inhibiting infection in the host with Zika Virus infection.

A method of treating a disease associated with Zika Virus infection that is comprised of administering one or more cardiac glycosides in an amount effective for inhibiting Zika Virus infection in the host with a Zika Virus infection is described but does not belong to the invention.

A drug is described but does not belong to the invention, wherein the drug is capable of curing a host of a Zika Virus infection, and potentially other types of arbovirus infections, by inhibiting the infection process.

A method of inhibiting Zika virus infection in a host is described but does not belong to the invention, wherein the method is comprised of administering one or more cardiac glycosides in an amount effective for inhibiting infection in the host with Zika Virus infection.

A method of treating a disease associated with Zika Virus infection is described but does belong to the invention, wherein the method is comprised of administering one or more cardiac glycosides in an amount effective for inhibiting Zika Virus infection in the host with a Zika Virus infection.

The cardiac glycosides are selected from the group consisting of oleandrin, digitoxin, ouabain, digoxin, β-methyl digitoxin, β-methyl digoxin, delanoside, lancoside C and proscillaridin or analogs thereof.

**Table 1. Structures of pyridinium compounds prepared for testing.**

| | | | |
|---|---|---|---|
| 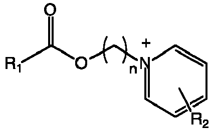 | 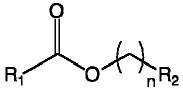 | | |
| | I⁻ or Br⁻ | | |
| | | | |
| | a. 1 - 20 | 21, 22 | |
| | | | |

| R₁ | Compound | n | IC₅₀ |
|---|---|---|---|
| 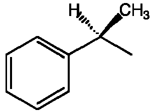 | 1, MRS 2572 | 4 | >30 |
| | 2, MRS 2573 | 6 | >30 |
| | 3, MRS 2481 | 8 | 1.81 ±0.58 |
| | 4, MRS 2574 | 10 | 2.52 0.39 |
| | | | |
| 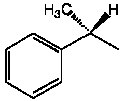 | 5, MRS 2485 | 8 | >25 |
| | | | |
| 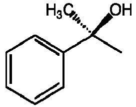 | 6, MRS 2515 | 8 | >30 |
| | | | |
| 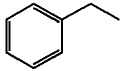 | 7, MRS 2480 | 8 | 12 ± 0.8 |
| | | | |
| 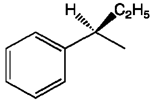 | 8, MRS 2591 | 8 | 3.16 ± 0.52 |
| | | | |
| 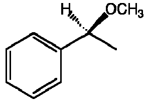 | 9, MRS 2506 | 8 | >30 |
| | | | |
| 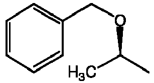 | 10, MRS 2507 | 8 | >30 |
| | | | |
| 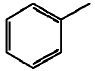 | 11, MRS 2513 | 8 | >30 |
| | | | |
| 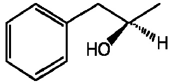 | 12, MRS 2514 | 8 | >30 |
| | | | |
| 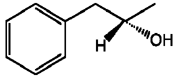 | 13, MRS 2516 | 8 | >30 |
| 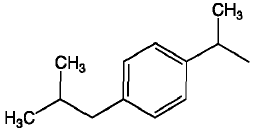 | 14, MRS 2590 | 8 | Toxic at 1 µM |
| | | | |
| 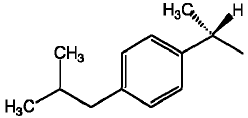 | 15, MRS 2390 | 8 | 2.2 ± 0.8 |
| | | | |
| 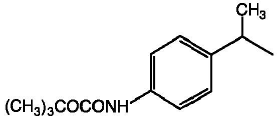 | 16, MRS 2517 | 8 | 4.6 ± 0.9 |
| | | | |
| 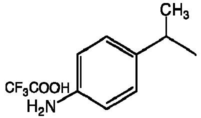 | 17, MRS 2518 | 8 | >30 |

### Compounds with R₂ ≠ H

| R₁ | Compound | R₂ = | IC₅₀ |
|---|---|---|---|
| 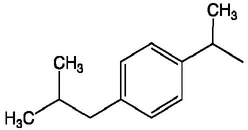 | 18, MRS 2589 | 3-CONH₂ | 5.56 ± 0.98 |
| 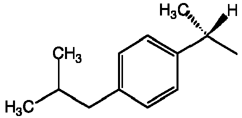 | 19, MRS 2421 | p-(CH₂)₂CH₃ | 3.3 ± 0.5 |
| | 20, MRS 2423 | p-(CH₂)₂-OH | 18 ± 0.9 |
| | 21, MRS 2422 | 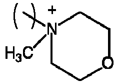 | 24 ± 1.0 |
| 22, MRS 2391 | 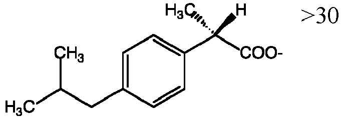 | | |

### BRIEF DESCRIPTION OF THE DRAWINGS

FIG. 1 shows the structures of Naproxen, Ibuprofen, MRS2481 and MRS2485; and,
FIG. 2 shows the structure of digitioxin.

### BRIEF DESCRIPTION OF THE TABLES

Table 1 illustrates the structures of the Amphiphilic pyridinium salts.
Table 2 illustrates the anticonvulsant properties of MES of MRS2481.
Table 3 illustrates the anticonvulsant properties of 6Hz of MRS2481.
Table 4 illustrates the anticonvulsant properties of 6Hz by MRS2485.
Table 5 illustrates that digitoxin suppresses expression of mRNA for AXL, *in vivo.*
Table 6 illustrates the digitoxin test on a rat model to treat epilepsy.

### DETAILED DESCRIPTION OF THE INVENTION

The following detailed descriptions are presented to enable a person skilled in the art to make and use the invention. For purposes of explanation, specific nomenclature is set forth to provide a thorough understanding of the present invention. However, it will be apparent to one skilled in the art that these specific details are not required to practice the invention. Descriptions of specific applications are provided only as representative examples. Various modifications in the preferred embodiments will be readily apparent to one skilled in the art, and the general principals defined herein may be applied to other embodiments and applications without departing from the scope of the invention. The present invention is not intended to be limited to the embodiments shown, but is to be accorded the widest possible scope consistent with the principals and features disclosed herein.

The pharmaceutical composition according to the invention includes an amphiphilic pyridinium compound selected from the group consisting of the compounds 1 to 20 of Table 1, wherein X- is acetate, mesylate, oxylate, chloride, bromide or iodide, and a pharmaceutically acceptable carrier, wherein the pharmaceutical composition is for use in treating epilepsy or related neurological disorders characterized by epileptic seizures in a mammal, wherein a therapeutically effective amount of the pharmaceutical composition is to be administered to the mammal.

In an embodiment that does not belong to the invention the amphiphilic pyridinium compounds of Table 1 are administered in combination with digitoxin to a mammal for the treatment of viral infections caused by EBOV or ZIKV.

In yet another embodiment that does not belong to the invention the amphiphilic pyridinium compounds of Table 1 are administered to a mammal for the treatment of epilepsy using one or more of the pyridinium salts shown in Table 1, in which X⁻ is an anion, such as iodide, bromide, acetate, halide, mesylate, oxylate, etc, to form an acceptable salt. Although not pyridinium salts and not belonging to the invention, compounds 21 (MRS 2422) and 22 (MRS 2391) are also disclosed herein.

A method for preventing epilepsy in a mammal by administering to a mammal a therapeutically effective amount of one or more amphiphilic pyridinium compound(s) of the present invention is described but does not belong to the invention. Administration of the amphiphilic pyridinium compound(s) may occur prior to the manifestation of symptoms characteristic of epilepsy, such that epilepsy is prevented, or alternatively, delayed in its progression.

The term "therapeutically effective amount," as used herein, is that amount that achieves at least partially a desired therapeutic or prophylactic effect in the brain. The amount of amphiphilic pyridinium compound necessary to bring about prevention and/or therapeutic treatment of epilepsy, or related condition, is not fixed *per se.* An effective amount is necessarily dependent upon the identity and the form of the pyridinium compound employed, the extent of the protection needed, or the severity of the epilepsy condition.

In conjunction with the prophylactic or therapeutic treatment, pharmacogenomics (i.e., the study of the relationship between an individual's genotype and the individual's response to a foreign compound or drug) may be considered. Differences in metabolism of therapeutics can lead to severe toxicity or therapeutic failure by altering the relation between dose and blood concentration of the pharmacologically active drug. Thus a physician or a clinician may consider applying knowledge obtained in relevant pharmacogenomics studies in determining whether to administer an amphiphilic pyridinium compound as well as tailoring the dosage and/or therapeutic regimen of treatment with an amphiphilic pyridinium compound.

Pharmacogenomics deals with clinically significant hereditary variations in the response to drugs due to altered drug disposition and abnormal action in affected persons. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These phamacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as a "genome-wide association," relies primarily on a high-resolution map of the human genome consisting of already known gene-related sites (e.g., a "bi-allelic" gene marker map which consists of 60,000-100,000 polymorphic or variable sites on the human genome, each of which has two variants). Such a high-resolution genetic map can be compared to a map of the genome of each of a statistically substantial number of subjects taking part in a Phase II/III drug trial to identify genes associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process. However, the vast majority of SNPs may not be disease associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals. Thus, mapping of the amphiphilic pyridinium compounds of the invention to SNP maps of patients may allow easier identification of these genes according to the genetic methods described herein.

Alternatively, a method termed the "candidate gene approach," can be utilized to identify genes that predict drug response. According to this method, if a gene that encodes a drug target is known, all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C19) has provided an explanation as to why some subjects do not obtain the expected drug effects or show exaggerated drug response and serious toxicity after taking the standard and safe dose of a drug. These polymorphisms are expressed in two phenotypes in the population, the extensive metabolizer and poor metabolizer. The prevalence of a poor metabolizer phenotypes is different among different populations. For example, the gene coding for CYP2D6 is highly polymorphic and several mutations have been identified in poor metabolizers, which all lead to the absence of functional CYP2D6. Poor metabolizers of CYP2D6 and CYP2C19 quite frequently experience exaggerated drug response and side effects when they receive standard doses. If a metabolite is the active therapeutic moiety, poor metabolizers show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification. Alternatively, a method termed the "gene expression profiling" can be utilized to identify genes that predict drug responses. For example, the gene expression of an animal dosed with a drug (e.g., in response to an amphiphilic pyridinium compound of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a mammal with an amphiphilic pyridinium compound.

The invention is further directed to pharmaceutical compositions comprising one or more amphiphilic pyridinium compound(s) of the present invention and a pharmaceutically acceptable carrier.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, hemectants, dispersion media, coatings, antibacterial or antifungal agents, isotonic and absorption delaying agents, penetration enhancers, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. The subcutaneous application can optionally be enhanced by co-administering a penetration enhancer, such as DMSO.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol.)

MRS2481 and its enantiomer MRS2485 have structures resembling the skeleton core of ibuprofen.

The EBOV is enveloped by a membrane, studded with glycoprotein trimers, which are themselves responsible for allowing the virus to make functional contact with target cells prior to penetration into the cell and productive infection. Therefore, any molecular strategy that could inactivate the membrane glycoprotein on the intact EBOV virion might be expected to be a candidate anti-EBOV drug. In a commonly used mouse model of EBOV infection, it has been reported that the anticancer drug toremifene and the non-steroidal anti-inflammatory drug ibuprofen are both inhibitors of EBOV infection. Nonetheless, the EBOV mouse model is not a very good model for EBOV infection in a human, and so it is not clear whether either of these specific compounds could be a lead for drug discovery.

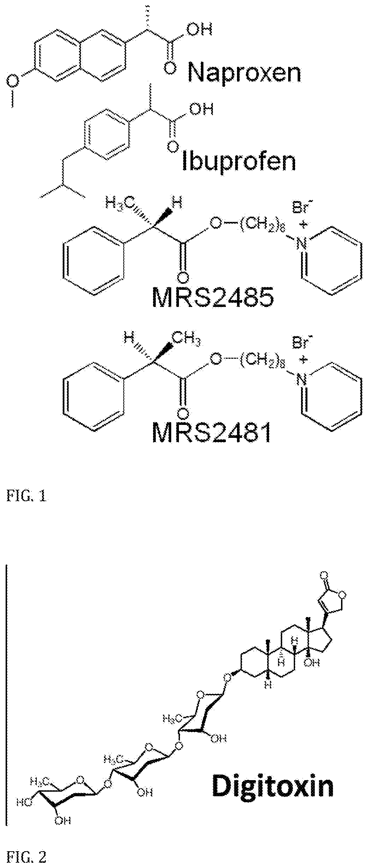

However, a high resolution X-Ray structure has been reported recently for the Ebola surface glycoprotein (Zhao Y et al, 2016), in which both toremifene and ibuprofen were found to bind to a common site. However, whether the molecular basis for prevention of infection in the mouse model is related to this common site of binding to the glycoprotein seems unlikely. In fact, the concentrations needed to actually affect the conformation of the pure glycoprotein are on the order of 100 µM (viz., 0.1 mM!) for toremifene, and >>100 mM for ibuprofen (see Figure 1, Zhao et al, 2016). Thus, these concentrations are at least 100 times the concentrations needed to block EBOV infection in the mouse model of EBOV infection. In addition, the pH needed to make these measurement is 5.2, and the pH of the body is close to 7.0, two orders of magnitude less acidic than the assay medium for the binding assay and for the stability of the glycoprotein crystal. Interestingly, though ibuprofen exists in two optical isoforms, S and R, and the S isoform is more active in the infection assay than the R isoform. However, both isoforms bind to the crystal structure of the glycoprotein, thus strongly adding to the conclusion that the EBOV glycoprotein binding site lacks specificity.

The two novel amphiphilic pyridinium compounds, MRS2481 and its enantiomer MRS2485, share a moiety with ibuprofen. It is expected that amphiphilic pyridinium compounds, in particular MRS2481 or its enantiomer MRS2485, will interfere with EBOV infection. It is proposed that these compounds can be considered leads for development small molecule inhibitors of EBOV infection.

The EBOV is enveloped by a glycoprotein trimer-laden membrane, and it is this glycoprotein which is responsible for allowing the virus to make contact with target cells prior to penetration into the cell and productive infection (Zhao et al, 2016). Therefore, any molecular strategy that could inactivate the membrane glycoprotein on the intact EBOV virion might be expected to be a candidate anti-EBOV drug. However, the ability of EBOV to attach and enter a cell is also dependent upon the activity of the tyrosine receptor kinase AXL at the cell surface (Shimojima et al, 2008). Thus a molecular strategy that suppressed expression of AXL in the host would also be an attractive anti-EBOV drug. Therefore, by mixing one drug against the membrane glycoprotein, with another against AXL, a new binary virocidal cocktail is envisioned, which could more efficiently treat or prevent EBOV infection than either compound alone. We therefore propose here to mix membrane glycoprotein inhibitor MRS2481, or its enantiomer MRS2485, with the AXL inhibitor digitoxin, to create a novel, small molecule anti-EBOV therapeutic. Since AXL is also required for ZIKV binding and penetration into target cells, the cocktail may also be useful for ZIKV infection. Digitoxin is also able to enter the brain (Flasch and Heinz, 1976; Rietbrock et al, 1977; Storstein et al, 1979), as does MRS2481/85, thus making the cocktail further relevant to the curious brain tropism of the Zika virus.

### MRS2481 and MRS2481 to inhibit the EBOV glycoprotein.

In a commonly used mouse model of EBOV infection, it has been reported that the anticancer drug toremifene and the non-steroidal anti-inflammatory drug ibuprofen are both inhibitors of EBOV infection. In the mouse EBOV model, ibuprofen exists in two optical isoforms, S and R: and the S isoform is more active in the infection assay than the R isoform. Consistently, a high resolution X-Ray structure has been reported recently for the Ebola surface glycoprotein (Zhao Y et al, 2016), in which both toremifene and ibuprofen were found to bind to a common site. Specifically, it has been reported that the anticancer drug toremifene blocked EBOV infection (*in vivo* IC50 ∼ 1 µM; Johansen et al, 2013). Recently, Zhao, et al, (2016) showed, in a high resolution X-Ray crystallography study, that the EBOV glycoprotein could binds toremifene (Kd = 16 uM), and also binds with very low affinity the nonsteroidal anti-inflammatory drug ibuprofen, in exactly the same site. (Kd = 6 mM). The test of ibuprofen was based on an *in silico* predicted interaction (Veljkovic et al, 2015). More recently, Johansen et al (2015) found that more activity could be found for naproxen (IC50 =6.2 uM; see FIG. 1) than for Ibuprofen. Thus, it has been described that a series of anti-inflammatory amphiphyllic pyridinium compounds, whose core structure includes the ibuprofen/naproxen skeleton (see **FIG. 1****).** Based on the modest binding constant for ibuprofen, and the many highly hydrophobic groups in the MRS 2481 and 2485, it is highly likely that either MRS2481 or MRS2485 or one of the other MRS compounds whose structure is shown can bind with greater potency and significantly destabilize the EBOV glycoprotein structure.

An additional property of these MRS2481/MRS2485 compounds is profound suppression of NFκB signaling, and reduction of IL-8 expression (Tchilibon et al, 2005). Relevantly, EBOV infection results in shedding of the glycoprotein into the circulation. Circulating EBOV glycoprotein binds to TLR4 (Toll like Receptor 4), which itself activates NFκB signaling through the MYT88/Ikkα/β pathway (Escudero-Perez et al 2014). This process of massive inflammation results in release of additional immune modulators, which in the additional presence of circulating EBOV glycoprotein, causes loss of blood vessel integrity and the classical phenotype of Ebola Hemorrhagic Fever (Escudero-Perez et al 2014). The additional immune modulators include IL-1β, TNFα, IL-6, MIP-1α and MIP1β (Baize et al, 2002). Thus, in addition to inhibiting EBOV glycoprotein activity, the MRS2481 and MRS2485 compounds may also block the ultimately lethal downstream hemorrhagic phenotype.

We have unexpectedly found that AXL mRNA is detectable in a castration resistant prostate cancer tumor (Pollard BS et al, submitted, 2016). The tumor was passaged in a syngeneic rat model with a normal immune system. The rats bearing the tumor were treated for 30 days with 0.03 mg/kg digitoxin. The drug was solubilized in 95% ethanol (ETOH), and diluted with phosphate buffered saline (PBS) prior to daily intraperitoneal (IP) injection. Messenger RNA was prepared from the resected tumors and measured by RNASeq on an Illumina GIIx sequencer. Table 5 shows that AXL mRNA is significantly reduced ca. 2-fold (p = 0.023, N = 8).

The inventor has unexpectedly found that AXL mRNA is detectable in a castration resistant prostate cancer tumor. The tumor was passaged in a syngeneic rat model with a normal immune system. The rats bearing the tumor were treated for 30 days with 0.03 mg/kg digitoxin. The drug was solubilized in 95% ethanol (ETOH), and diluted with phosphate buffered saline (PBS) prior to daily intraperitoneal (IP) injection. Messenger RNA was prepared from the resected tumors and measured by RNASeq on an Illumina GIIx sequencer.

In addition to blocking AXL expression, thereby inhibiting EBOV infection, digitoxin also has profoundly potent anti-inflammatory activity. As for the MRS2481.85 compounds, this activity also depends mechanistically on suppression the TNFα/NFκB signaling pathway (Srivastava et al, 2004; Pollard et al, *submitted,* 2016). A cocktail of MRS2481/85 and digitoxin ("MD Cocktail") would thus suppress both EBOV glycoprotein function and downstream lethal proinflammatory signaling. The anti-inflammatory property of the MRS2481/MRS2485 compounds is based on profound suppression of NFκB signaling, and reduction of IL-8 expression (Tchilibon et al, 2005). Relevantly, EBOV infection results in shedding of the glycoprotein into the circulation. Circulating EBOV glycoprotein binds to TLR4 (Toll like Receptor 4), which itself activates NFκB signaling through the MYT88/Ikkα/β pathway (Escudero-Perez et al 2014). This process of massive inflammation results in release of additional immune modulators, which in the additional presence of circulating EBOV glycoprotein, causes loss of blood vessel integrity and the classical phenotype of Ebola Hemorrhagic Fever (Escudero-Perez et al 2014). The additional NFκB-driven immune modulators include IL-1β, TNFα, IL-6, MIP-1α and MIP1β (Baize et al, 2002). Thus in addition to inhibiting EBOV glycoprotein activity, the MRS2481/85 compounds may also block the ultimately lethal downstream hemorrhagic phenotype.

The MRS2481/85 and digitoxin cocktail may also suppress Zika Virus and other arbovirus infection.

Zika Virus (ZIKV) is the first mosquito-transmitted arbovirus shown to cause fetal abnormalities and microcephaly in unborn babies. Adults develop fever, rash, arthralgia and conjunctivitis, and can also develop Guillain-Barre syndrome. A candidate viral entry receptor, AXL, from the TAM phosphatidylserine receptor family, has been identified in the developing brain, and is highly expressed by radial glial cells, astrocytes, endothelial cells and microglia (Nowakowski TJ, et al, 2016). High levels of AXL expression are conserved in developing mouse and ferret cortex (Nowakowski et al, 2016). Cancer cells also express high levels of AXL, including breast (Meric et al, 2002), lung (Wimmel et al, 2001; Hamel et al, 2015), lymphomas and leukemias (Challier et al, 1996), colon (Craven et al, 1995), and prostate (Bansal et al, 2015).

The mosquito takes advantage of the fact that skin is a convenient site of entry for both ZIKV and dengue virus (Hamel et al, 2015). There, AXL is also the major candidate viral entry receptor in dermal fibroblasts, epidermal keratinocytes, immature dendritic cells, and lung epithelial cells. (Hamel R et al, 2015). Other minor candidate Zika receptors have been identified in a subset of cells from the epidermis, including adhesion factors DC-SIGN, TYRO3, and TIM-1 (Hamel et al, 2015). TIM-1 is another commonality, as it is also an attachment factor for EBOV (Yuan, et al, 2015).

Except for Yellow Fever, there are no commercial vaccines presently available for Zika, Dengue Fever or other arbovirus infections. However, vaccines are effective only if the host has been vaccinated. A drug to prevent entry of ZIKV, or other arbovirus, into the host would treat those who were not vaccinated. Furthermore, there is a developing concern as to whether a previous infection with, or vaccinated against, one flavivirus might mediate antibody-dependent enhancement of a secondary severe infection (Haug et al, 2016). Therefore, a targeted antiviral against the entry mechanism for ZIKV would have additional advantages.

By mixing one drug against the membrane glycoprotein, with another against AXL, a new binary virocidal cocktail can be created which could more efficiently treat or prevent EBOV infection than either compound alone. In addition, realizing that ZIKV also depends on AXL for entering target cells, we suggest that the cocktail may also be useful for treating or preventing ZIKV infection. The fact that digitoxin enters the brain suggests that this part of the cocktail might also suppress the microcephaly phenotype noted recently for ZIKV infection. We therefore propose here to mix membrane glycoprotein inhibitor MRS2481, or its enantiomer MRS2485, with the AXL inhibitor digitoxin, to create a novel, small molecule anti-EBOV/ anti-ZIKV therapeutic.

Cardiac glycosides inhibit Na⁺-K⁺ATPase at high concentrations, and function as inhibitors of TNFα-NFκB signaling (Srivastava M, et al, 2004; Yang et al, 2005) and NFAT-cMYC signaling (Yang et al, 2013) at lower concentrations. Of the digitalis drugs tested in this disclosure, only digitoxin and oleandrin are able to function as inhibitors of TNFα-NFκB signaling at concentrations that are not also toxic to man

In the present invention the term arbovirus is used to encompass Zika virus, Dengue Virus, Japanese Encephalitis Virus, Rift Valley Fever Virus, Tick-Born Encephalitis Virus. West Nile Virus, and Yellow Fever Virus. Therefore, the method of the present invention can treat infections caused by these arboviruses.

In the method of the present invention, the active components, i.e., the cardiac glycosides, may be prepared in a variety of forms depending on the kind of cardiac glycoside and how it is used. The forms and administration routes may be the same as those employed in the use of commercial cardiac glycosides. For example, in the case of digitoxin, it is usually administered as a pill that is swallowed, but it can be given sublingually. Alternatively, it can be prepared for injection as a liquid, an emulsion or a suspension.

The dose of the aforementioned pharmaceutical agent varies depending on the manner of administration, age, sex, and other conditions of the patient, including the disease and the severity of disease. Usually the amount of the effective component, for example of digitoxin, can range between 0.01 to 0.2 mg/day.

### EXAMPLES

EXAMPLE 1. Demonstration that the Amphiphilic pyridinium salt MRS2481 mitigates a model of epilepsy.

### 1.1(a) First test for MRS2481 on the animal model:

The model used is the Maximal Electro-Shock (MES) test in mice. Briefly, the MES is a model for generalized tonic-clonic seizures and provides an indication of a compound's ability to prevent seizure spread when all neuronal circuits in the brain are maximally active. These seizures are highly reproducible and are electrophysiologically consistent with human seizures. For all tests based on MES convulsions, 60Hz of alternating current (50 mA in mice and 150 mA in rats) was delivered for 0.2s by corneal electrodes which have been primed with an electrolyte solution containing an anesthetic agent (0.5% tetracaine HCl). Mice or rats were tested at various intervals following doses of 30, 100 and 300 mg/kg of test compound given by i.p. injection or through oral dosing (p.o.).Other doses can be used if indicated by previously known pharmacology or to determine an ED₅₀. An animal was considered "protected" from MES-induced seizures upon abolition of the hindlimb tonic extensor component of the seizure (Swinyard et al., 1989; White et al., 1995a; White et al., 1995b).

Conditions and controls: Compounds were injected into mice at 30 and 100 mg/kg, and assayed at 30 minutes.

**Table 2. Protection of animals from convulsant MES by MRS2481**

| Dose | Time: 30 minutes |
|---|---|
| | |
| 100 mg/kg | 1 of 4 animals protected |

### 1.2.1 (a) Second type of animal model test for MRS2481:

Some clinically useful AEDs are ineffective in the standard MES and scMET tests but still have anticonvulsant activities *in vivo.* In order to identify potential AEDs with this profile, compounds may be tested in the minimal clonic seizure (6Hz or 'psychomotor') test (Barton et al., 2001). Like the maximal electroshock (MES) test, the minimal clonic seizure (6Hz) test is used to assess a compound's efficacy against electrically induced seizures but uses a lower frequency (6Hz) and longer duration of stimulation (3s). Test compounds were pre-administered to mice via i.p. injection. At varying times, individual mice (four per time point) were challenged with sufficient current delivered through corneal electrodes to elicit a psychomotor seizure in 97% of animals (32 mA for 3s) (Toman et al., 1952). Untreated mice displayed seizures characterized by a minimal clonic phase followed by stereotyped, automatistic behaviors described originally as being similar to the aura of human patients with partial seizures. Animals not displaying this behavior are considered protected. The test was evaluated quantitatively by measuring the responses at varying doses at a determined time of peak effect (TPE).

1.2 (b) Conditions and controls: Compounds were injected into mice at 100 mg/kg, and assayed at 30 minutes.

**Table 3. Protection from convulsant 6Hz by MRS2481**

| DOSE | Time: 30 Minutes |
|---|---|
| | |
| 100 mg/kg | 2 out of 4 animals protected |

EXAMPLE 2. Demonstration on second compound that shows the Amphiphilic pyridinium salt MRS2485 mitigates a model of epilepsy.

2.1(a) animal model: Some clinically useful AEDs are ineffective in the standard MES and scMET tests but still have anticonvulsant activities *in vivo.* In order to identify potential AEDs with this profile, compounds may be tested in the minimal clonic seizure (6Hz or 'psychomotor') test (Barton et al., 2001). Like the maximal electroshock (MES) test, the minimal clonic seizure (6Hz) test is used to assess a compound's efficacy against electrically induced seizures but uses a lower frequency (6Hz) and longer duration of stimulation (3s). Test compounds were pre-administered to mice via i.p. injection. At varying times, individual mice (four per time point) were challenged with sufficient current delivered through corneal electrodes to elicit a psychomotor seizure in 97% of animals (32 mA for 3s) (Toman et al., 1952). Untreated mice displayed seizures characterized by a minimal clonic phase followed by stereotyped, automatistic behaviors described originally as being similar to the aura of human patients with partial seizures. Animals not displaying this behavior are considered protected. The test was evaluated quantitatively by measuring the responses at varying doses at a determined time of peak effect (TPE).

Conditions and controls; Compounds were injected into mice at 30 and 100 mg/kg, and assayed at 30 minutes.

**Table 4. Protection of animals from convulsant 6Hz by MRS2485**

| Dose | Time: 30 minutes |
|---|---|
| 30 mg/kg | 4 of 4 animals protected |
| 100 mg/kg | 4 of 4 animals protected |

Example 3. Digitoxin was administered for 30 days *in vivo* to rats that had prostate cancer growing in them. The tumors were excised and evaluated for mRNA on an Illumina platform. The digitoxin dose administered is compatible with human life. The digitoxin treatment lowered the AXL gene messengerRNA (mRNA) production two-fold. This is relevant to both Zika and Ebola virus.

**Table 5**

| **AXL mRNA, FPKM*** | **Digitoxin, 0.03 mg/kg, for 30 days** | **p value,** 2 tailed | **N** | **Fold Change** |
|---|---|---|---|---|
| **28.2** | **-** | **-** | **4** | **-** |
| **14.2** | **+drug** | **0.023** | **4** | **-1.99** |
| **Table 5. Digitoxin blocks expression of AXL mRNA expression.** *FPKM, Fractional Reads per Million Reads. Data collected by next generation sequencing on an Illumina platform. | | | | |

| | | | | |
|---|---|---|---|---|
| *FPKM, Fractional Reads per Million Reads | | | | |

Example 4. Digitoxin was tested on a rat model as a treatment for epilepsy. Eight animals were tested. The animal weight range was: 100.0 - 155.0 g

**Table 6**

| Test | Dose (mg/kg) | Time (hrs) | Dths | N/F C |
|---|---|---|---|---|
| MES | 0.03 | 0.5 | | 0/8 |
| MES | 0.3 | 0.5 | | 0/8 |
| SCMET | 0.03 | 0.5 | | 1/8 |
| SCMET | 0.3 | 0.5 | | 0/8 |

Each animal weighed and dosed for four days and tested 30 min after last day's dose. No toxicity observed

### REFERENCES

Baize S, Leroy EM, Georges AJ, Georges-Courbot MC, Capron M, Bedjabaga I, Lansoud-Soukate J, Mavoungou E.,Inflammatory responses in Ebola virus-infected patients. Clin.Exptl Immunol. 128:163-168, 2002.
Bansal N, Mishra PJ, Stein M, DiPaola RS and Bertino JR AXL receptor tyrosoine kinase is upregulated in metformin resistant prostate cancer cells. Oncotarget 6:15321-15331, 2015.
Challier C, Uphoff CC, Janssen JW, Drexler HG, Differential expression of the ufo/axl oncogene in human leukemia-lymphoma cell lines Leukemia 10:781-787, 1996.
Choi J, Min HJ, Shin JS. Increased levels of HMGB1 and pro-inflammatory cytokines in children with febrile seizures. J Neuroinflammation. 2011 Oct 11;8:135. doi: 10.1186/1742-2094-8-135. PMID: 21989210 [PubMed - indexed for MEDLINE].
Craven RJ, Xu LH, Weiner TM, Fridell YW, Dent GA, Srivastava S, Varnum B, Liu ET, Cance WG. Receptor Tyrosine kinases expressed in metastatic colon cancer Int.J.Cancer 60:791-797,1995.
De Herdt V, Bogaert S, Bracke KR, Raedt R, De Vos M, Vonck K, Boon P. Effects of vagus nerve stimulation on pro- and anti-inflammatory cytokine induction in patients with refractory epilepsy. J Neuroimmunol. 2009 Sep 29;214(1-2):104-8. doi: 10.1016/j.jneuroim.2009.06.008. Epub 2009 Jul 15.
Diaz JC, Simakova O, Jacobson KA, Arispe N, Pollard HB. Small molecule blockers of the Alzheimer Abeta calcium channel potently protect neurons from Abeta cytotoxicity. Proc Natl Acad Sci U S A. 2009 Mar 3;106(9):3348-53. doi: 10.1073/pnas.0813355106. Epub 2009 Feb 9.
Devinsky O, Vezzani A, et al. Glia and epilepsy: excitability and inflammation. Trends in Neurosciences 36:174-184, 2013.
During MJ and Spencer DD Extracellular hippocampal glutamate and spontaneous seizure in the conscious human brain. Lancet 341:1607-1610,1993.
Eisenstein M. Unrestrained Excitement Nature 511: S4-S6, 2014.
Fauci AS and Moren DM, Zika virus in the Americas-yet another arbovirus threat. New Eng. J.Med. 374: 601-603, 2016.
Hamel R, Dejarnac O, Wichit S, Ekchariyawat P, Neyret A, Lupiertiop N, Perea-Lecoin M, Surasombatpattana P, Talignani L, Thomas F, Cao-Lormeau V-M, Choumet V, Briant L, Despres P, Amara A, Yssel H and Misse D, Biology of Zika Virus infection in human skin cells. J. Virol. 89:8880-8896, 2015.
Haug CJ, Kieny MP and Murgue B. The Zika Challenge. New Eng. J. Med. 374:1801-1803, 2016.
Johansen LM, Brannan JM, Delos SE, Shoemaker CJ, Stossel A, Lear C, Hoffstrom BG, Dewald LE, Schornberg KL, Scully C, Lehár J, Hensley LE, White JM, Olinger GG. FDA-approved selective estrogen receptor modulators inhibit Ebola virus infection. Sci Transl Med. 5(190):190ra79. doi: 10.1126/scitranslmed.3005471, 2013.
Johansen LM, DeWald LE, Shoemaker CJ, Hoffstrom BG, Lear-Rooney CM, Stossel A, Nelson E, Delos SE, Simmons JA, Grenier JM, Pierce LT, Pajouhesh H, Lehár J, Hensley LE, Glass PJ, White JM, Olinger GG. A screen of approved drugs and molecular probes identifies therapeutics with anti-Ebola virus activity. Sci Transl Med. 2015 Jun 3;7(290):290ra89. doi: 10.1126/scitranslmed.aaa5597, 2015.
Laurén HB1, Lopez-Picon FR, Brandt AM, Rios-Rojas CJ, Holopainen IE. Transcriptome analysis of the hippocampal CA1 pyramidal cell region after kainic acid-induced status epilepticus in juvenile rats. PLoS One. 2010 May 20;5(5):e10733. doi: 10.1371/journal.pone.0010733.
Librizzi L, et al. Seizure induced brain-born inflammation sustains seizure recurrence and blood-brain-barrier damage. Ann. Neurol. 72:82-90, 2012).
Meric F, Lee WP, Sahin A, Zhang H, Kung HJ, and Hung MC. Expression profile of tyrosine kinases in breast cancer. Clin. Cancer Res. 8:361-367, 2002.
Nowakowski TJ, Pollen AA, Lullo ED, Sandoval-Espinoza C, Bershteyn M, and Kriegstein AR. Expression analysis highlights AXL as a candidate Zika Virus Entry Receptor in neural stem cells. Cell Stem Cell 18:1-6, 2016.
Pollard JR, Eidelman O, Mueller GP, Dalgard CL, Crino PB, Anderson CT, Brand EJ, Burakgazi E, Ivaturi SK, Pollard HB. The TARC/sICAM5 Ratio in Patient Plasma is a Candidate Biomarker for Drug Resistant Epilepsy. Front Neurol. 2013 Jan 3;3:181. doi: 10.3389/fneur.2012.00181. eCollection 2012. PMID: 23293627.
Rietbrock N, Kuhlmann J, Vohringer HF Pharmakokinetik von Herz-glykosiden und klinische Konsequenzen. Fortschr.Med. 95:909-915 & 951-954, 1977.
Shimojima M, Ikeda Y, Kawaoka Y, The mechanism of Axl-Mediated Ebola Virus Infection. J.Inf.Dis. 2007:196 (Suppl 2. S259-263) 2007.
Srivastava M, Digitoxin mimics gene therapy with CFTR and suppresses hypersecretion of IL-8 from cystic fibrosis lung epithelial cells. Proc.Nat.Acad.Sci. (USA), 2004.
Storstein L, Nore AK and Sjaastad O. Studies on digitalis. 23. Blood brain barrier of digitoxin in humans, Clin.Cardiol. 2:146-50, 1979.
Swinyard EA, Woodhead JH, White HS and Franklin MR (1989) General principles: experimental selection, quantification, and evaluation of anticonvulsants, in Antiepileptic Drugs (R.H.Levy RHM, B. Melrum, J.K. Penry and F.E. Dreifuss ed) pp 85-102, Raven Press, New York.
Tchilibon S, Zhang J, Yang Q, Eidelman O, Kim H, Caohuy H, Jacobson KA, Pollard BS, Pollard HB. Amphiphilic pyridinium salts block TNF alpha/NF kappa B signaling and constitutive hypersecretion of interleukin-8 (IL-8) from cystic fibrosis lung epithelial cells. Biochem Pharmacol. 2005 Aug 1;70(3):381-93.PMID: 15963954 [PubMed - indexed for MEDLINE].
Veljkovic V, Goeijenbier M, Glisic S, Veljkovic N, Perovic VR, Sencanski M, Branch DR, Paessler S. In silico analysis suggests repurposing of ibuprofen for prevention and treatment of EBOLA virus disease. F1000Res. 2015 May 1;4:104. doi: 10.12688/f1000research.6436.1. eCollection 2015.
White HS, Johnson M, Wolf HH and Kupferberg HJ (1995a) The early identification of anticonvulsant activity: role of the maximal electroshock and subcutaneous pentylenetetrazol seizure models. Ital J Neurol Sci 16:73-7.
White HS, Woodhead JH and Franklin MR (1995b) General principles: experimental selection, quantification, and evaluation of antiepileptic drugs, in Antiepileptic Drugs (Levy RHM, R.H.; Meldrum, B.S. ed) pp 99-110, Raven Press, New York.
Wimmel A, Glitz D, Kraus A, Roeder J, Schuermann M. Axl receptor tyrosine kinase expression in human lung cancer cell lines correlates with cellular adhesion. Eur. J. Cancer 37: 2264-2274. 2001.
Yang Q, Huang W, Jozwik C, Lin Y, Glasman M, Caohuy H, Srivastava M, Esposito D, Gillette W, Hartley J, Pollard HB. Cardiac glycosides inhibit TNF-alpha/NF-kappaB signaling by blocking recruitment of the TNF receptor-associated death domain to the TNF receptor. Proc.Nat.Acad.Sci. (USA), 102: 9631-9636, 2005.
Yang Q, Dalgard CL, Eidelman O, Jozwik C, Pollard BS Srivastava M, and Pollard HB Digitoxin induces apoptosis in cancer cells by inhibiting nuclear factor of activated T-cell-driven c-MYC expression. J. Carcinogenesis 12:8.doi:10.4102/1477-3163.112268.Print 2013.
Youn YA1, Kim SJ, Sung IK, Chung SY, Kim YH, Lee IG. Serial examination of serum IL-8, IL-10 and IL-IRa levels is significant in neonatal seizures induced by hypoxic-ischaemic encephalopathy Scand J Immunol. 2012 Sep;76(3):286-93. doi: 10.1111/j.1365-3083.2012.02710.x.
Zhao Y, Ren J, Harlos K, Jones DM, Zeltina A, Bowden TA, Padilla-Parra S, Fry EE and Stuart DI. Toremifene interacts with and destabilizes the Ebola virus glycoprotein. Nature 535(7610): 169-172, 2016.

## Claims

1. A pharmaceutical composition including:
an amphiphilic pyridinium compound selected from the group consisting of 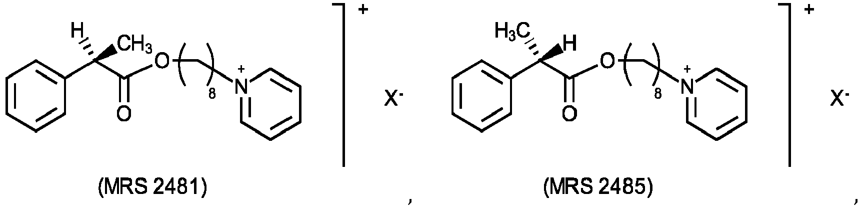 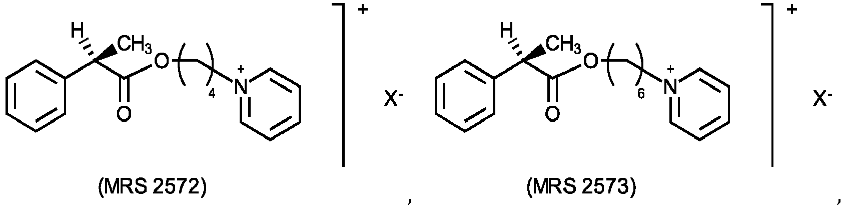 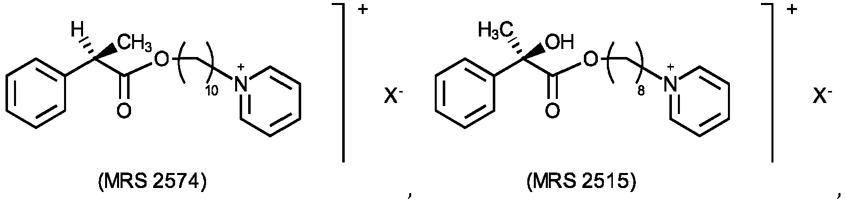 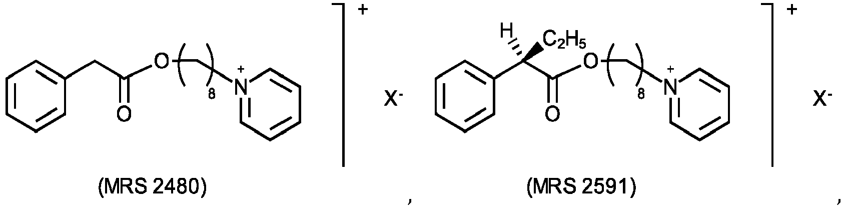 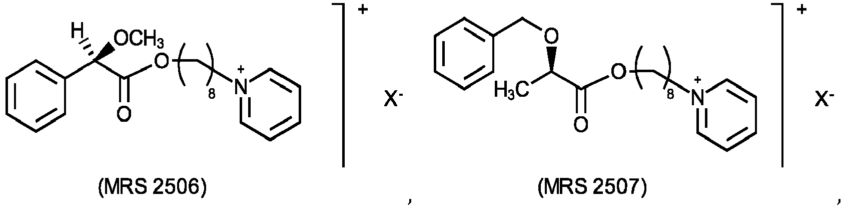 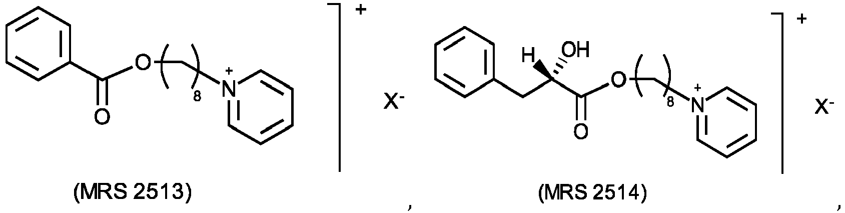 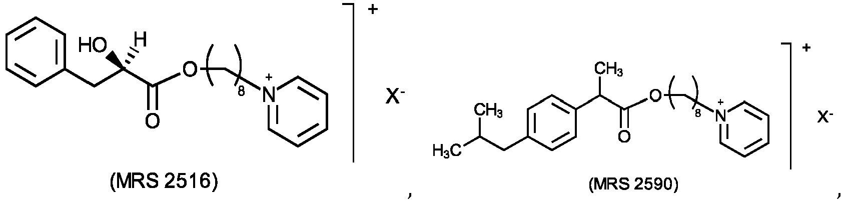 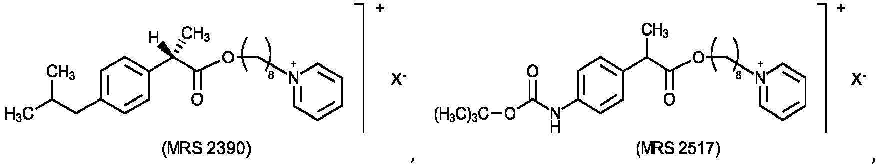 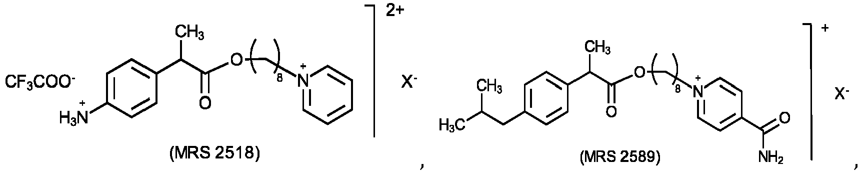 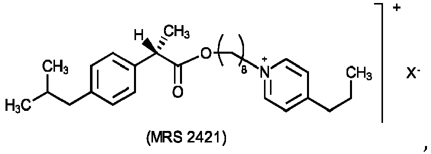 and 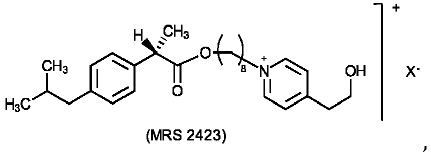
wherein X⁻ is acetate, mesylate, oxylate, chloride, bromide or iodide, and
a pharmaceutically acceptable carrier
for use in treating epilepsy or related neurological disorders **characterized by** epileptic seizures in a mammal, wherein a therapeutically effective amount of the pharmaceutical composition is to be administered to the mammal.

2. The pharmaceutical composition for use according to claim 1, wherein the amphiphilic pyridinium compound is

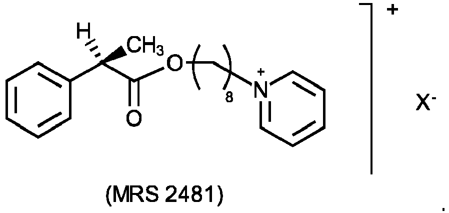

3. The pharmaceutical composition for use according to claim 1, wherein the amphiphilic pyridinium compound is

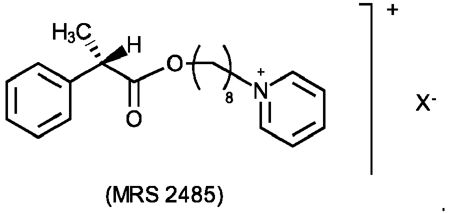

4. The pharmaceutical composition for use of claim 1, wherein the disease to be treated is epilepsy.

5. The pharmaceutical composition for use of claim 1, wherein the pharmaceutical composition is to be administered orally.

6. The pharmaceutical composition for use of claim 1, wherein the pharmaceutical composition is to be administered intravascularly.

7. The pharmaceutical composition for use of claim 1, wherein the pharmaceutical composition is to be administered intramuscularly.

8. The pharmaceutical composition for use of claim 1, wherein the pharmaceutical composition is to be administered subcutaneously.

9. The pharmaceutical composition for use of claim 8, wherein the pharmaceutical composition is to be administered with a penetration enhancer.

10. The pharmaceutical composition for use of claim 1, wherein the pharmaceutical composition is to be administered intraperitoneally.

11. The pharmaceutical composition for use of claim 1, wherein the pharmaceutical composition is to be administered prior to the manifestation of symptoms of epilepsy.

## Patentansprüche

1. Eine pharmazeutische Zusammensetzung aufweisend:
eine amphiphile Pyrdiniumverbindung ausgewählt aus der Gruppe bestehend aus 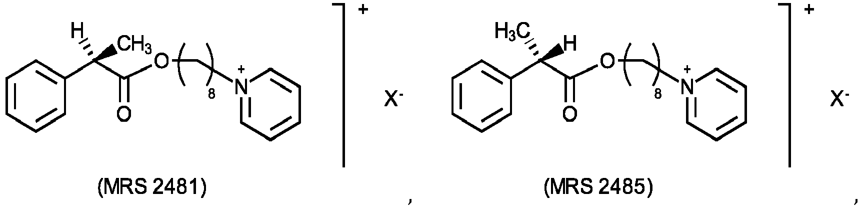 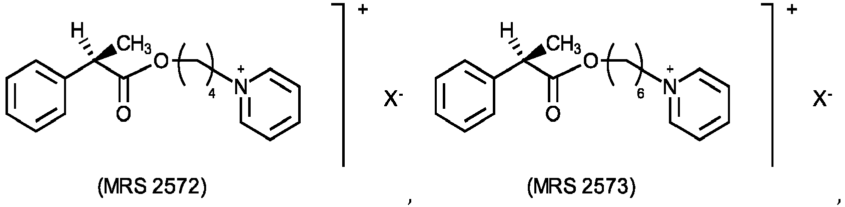 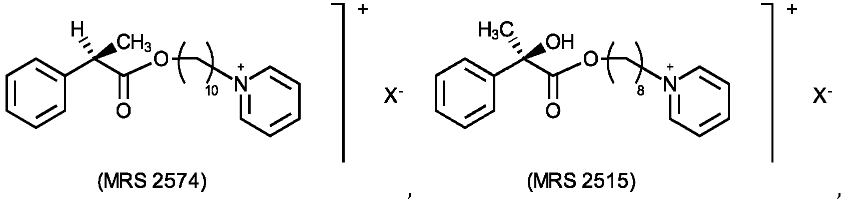 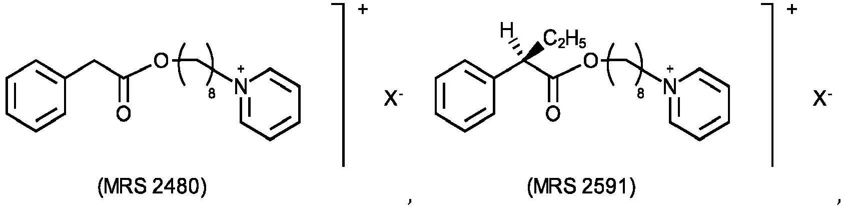 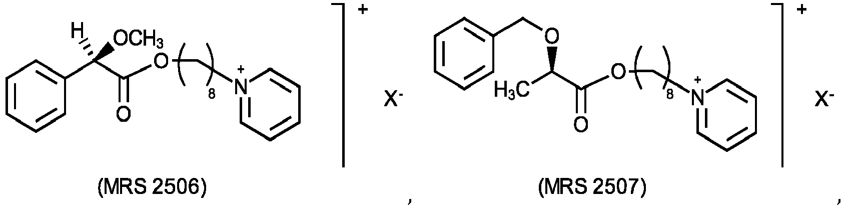 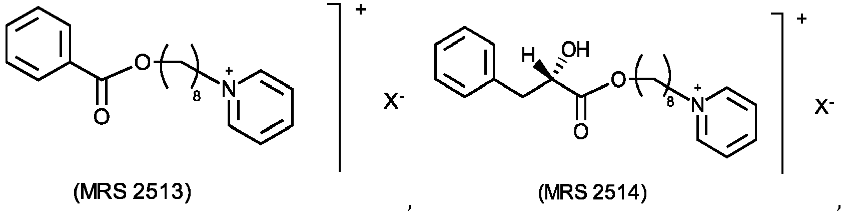 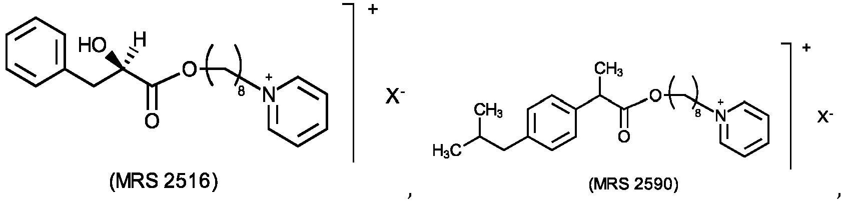 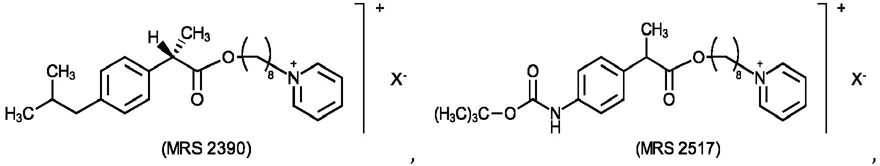 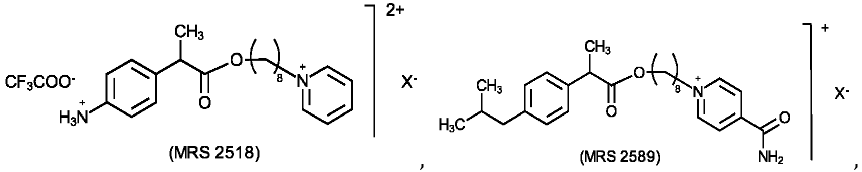 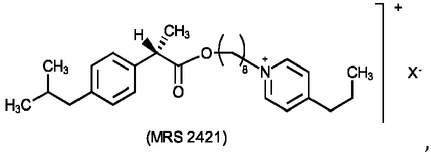 und 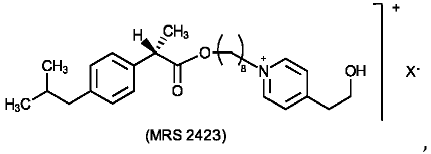
wobei X⁻ Acetat, Mesylat, Oxalat, Chlorid, Bromid oder Iodid ist, und
ein pharmazeutisch akzeptierbarer Träger
zur Verwendung bei der Behandlung von Epilepsie oder verwandter neurologischer Störungen, die durch epileptische Anfälle in einem Säugetier gekennzeichnet sind, wobei eine therapeutisch effektive Menge der pharmazeutischen Zusammensetzung dem Säugetier zu verabreichen ist.

2. Pharmazeutische Zusammensetzung zur Anwendung gemäß Anspruch 1, wobei die amphiphile Pyrdiniumverbindung 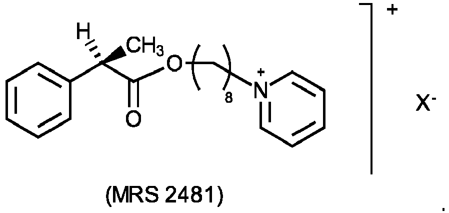 ist.

3. Pharmazeutische Zusammensetzung zur Anwendung gemäß Anspruch 1, wobei die amphiphile Pyrdiniumverbindung 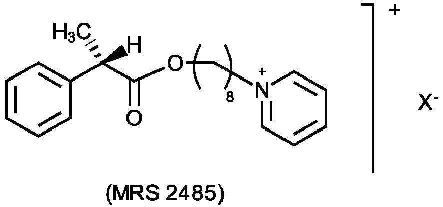 ist.

4. Pharmazeutische Zusammensetzung zur Anwendung gemäß Anspruch 1, wobei die zu behandelnde Krankheit Epilepsie ist.

5. Pharmazeutische Zusammensetzung zur Anwendung gemäß Anspruch 1, wobei die pharmazeutische Zusammensetzung oral zu verabreichen ist.

6. Pharmazeutische Zusammensetzung zur Anwendung gemäß Anspruch 1, wobei die pharmazeutische Zusammensetzung intravaskulär zu verabreichen ist.

7. Pharmazeutische Zusammensetzung zur Anwendung gemäß Anspruch 1, wobei die pharmazeutische Zusammensetzung intramuskulär zu verabreichen ist.

8. Pharmazeutische Zusammensetzung zur Anwendung gemäß Anspruch 1, wobei die pharmazeutische Zusammensetzung subkutan zu verabreichen ist.

9. Pharmazeutische Zusammensetzung zur Anwendung gemäß Anspruch 1, wobei die pharmazeutische Zusammensetzung mit einem Penetrationsförderer zu verabreichen ist.

10. Pharmazeutische Zusammensetzung zur Anwendung gemäß Anspruch 1, wobei die pharmazeutische Zusammensetzung intraperitoneal zu verabreichen ist.

11. Pharmazeutische Zusammensetzung zur Anwendung gemäß Anspruch 1, wobei die pharmazeutische Zusammensetzung vor der Manifestation von Symptomen der Epilepsie zu verabreichen ist.

## Revendications

1. Composition pharmaceutique comprenant :
un composé de type pyridinium amphiphile choisi dans le groupe constitué par 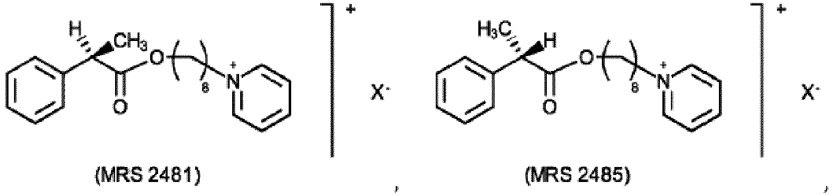 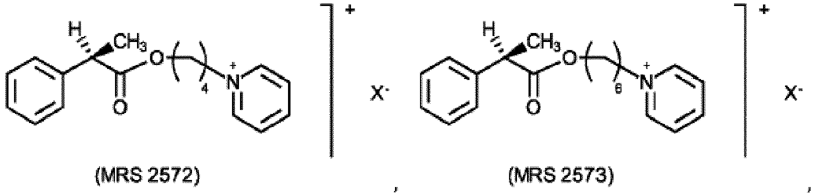 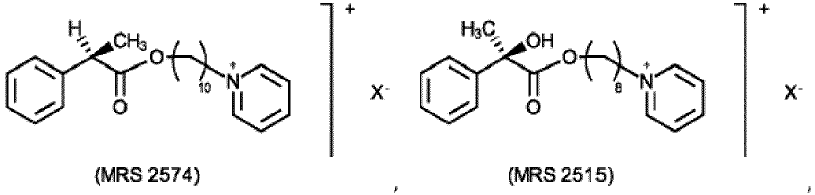 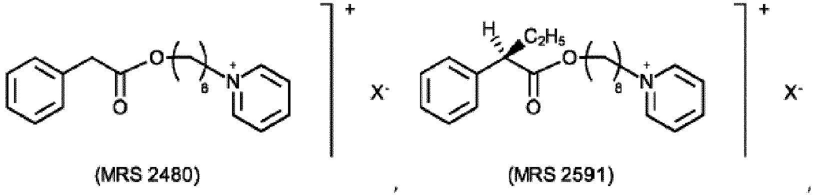 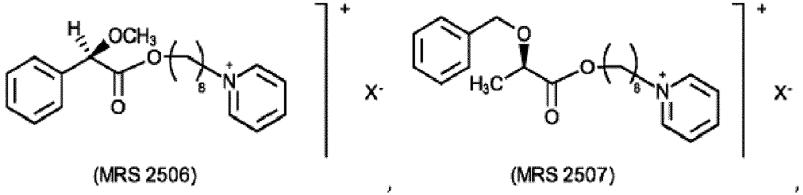 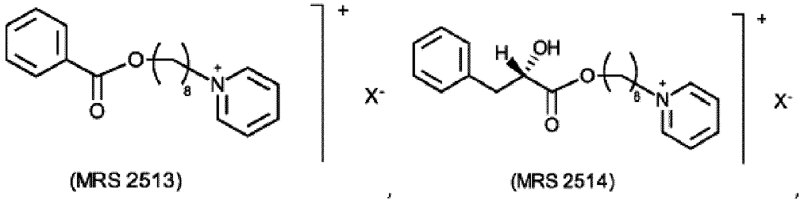 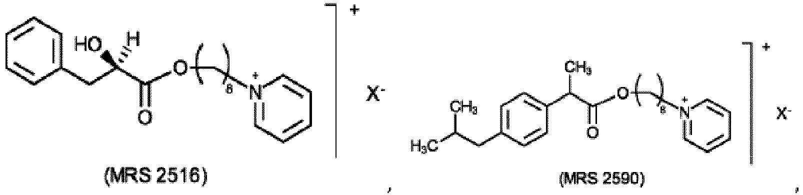 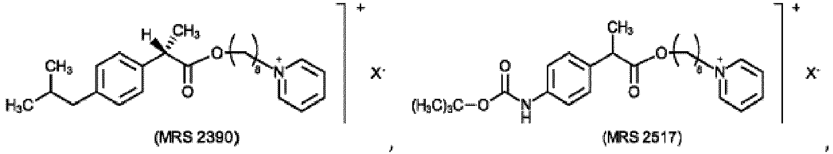 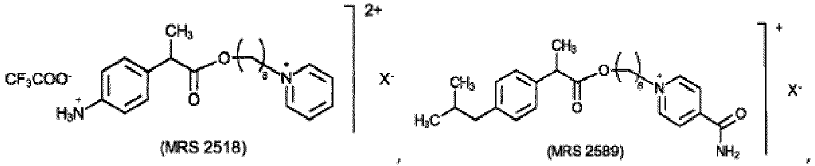 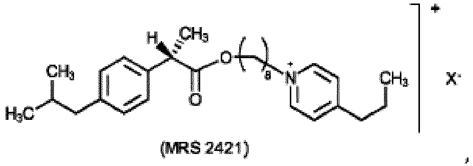 et 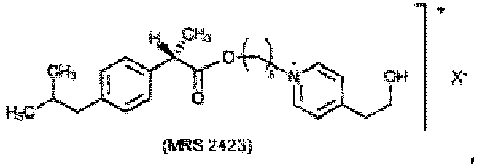
X⁻ étant acétate, mésylate, oxylate, chlorure, bromure ou iodure, et
un support pharmaceutiquement acceptable
pour une utilisation dans le traitement de l'épilepsie ou de troubles neurologiques liés **caractérisés par** des crises épileptiques chez un mammifère, une quantité thérapeutiquement efficace de la composition pharmaceutique devant être administrée au mammifère.

2. Composition pharmaceutique pour une utilisation selon la revendication 1, le composé de type pyridinium amphiphile étant

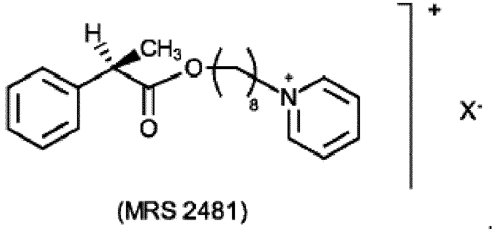

3. Composition pharmaceutique pour une utilisation selon la revendication 1, le composé de type pyridinium amphiphile étant

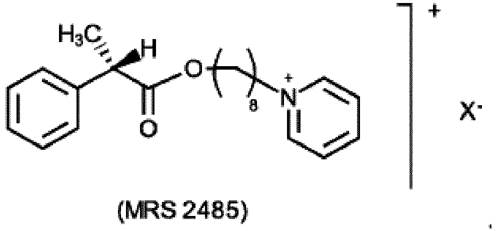

4. Composition pharmaceutique pour une utilisation selon la revendication 1, la maladie devant être traitée étant l'épilepsie.

5. Composition pharmaceutique pour une utilisation selon la revendication 1, la composition pharmaceutique devant être administrée de manière orale.

6. Composition pharmaceutique pour une utilisation selon la revendication 1, la composition pharmaceutique devant être administrée de manière intravasculaire.

7. Composition pharmaceutique pour une utilisation selon la revendication 1, la composition pharmaceutique devant être administrée de manière intramusculaire.

8. Composition pharmaceutique pour une utilisation selon la revendication 1, la composition pharmaceutique devant être administrée de manière sous-cutanée.

9. Composition pharmaceutique pour une utilisation selon la revendication 8, la composition pharmaceutique devant être administrée avec un amplificateur de pénétration.

10. Composition pharmaceutique pour une utilisation selon la revendication 1, la composition pharmaceutique devant être administrée de manière intrapéritonéale.

11. Composition pharmaceutique pour une utilisation selon la revendication 1, la composition pharmaceutique devant être administrée avant la manifestation de symptômes d'épilepsie.
